(19) Europäisches Patentamt / European Patent Office / Office européen des brevets

(11) **EP 4 154 808 B1**

(12) **EUROPÄISCHE PATENTSCHRIFT**

(45) Veröffentlichungstag und Bekanntmachung des Hinweises auf die Patenterteilung:
**18.03.2026  Patentblatt 2026/12**

(21) Anmeldenummer: **22194547.0**

(22) Anmeldetag: **08.09.2022**

(51) Internationale Patentklassifikation (IPC):
**A61B 5/03** *(2006.01)*        **A61B 5/00** *(2006.01)*
**A61B 5/08** *(2006.01)*

(52) Gemeinsame Patentklassifikation (CPC):
**A61B 5/037; A61B 5/0803; A61B 5/6853;**
A61B 2505/03; A61B 2560/0276; A61B 2562/0247

(54) **SYSTEM ZUR BEFÜLLUNG EINES ÖSOPHAGUSBALLONS**

SYSTEM FOR FILLING AN ESOPHAGEAL BALLOON

SYSTÈME DE REMPLISSAGE D'UN BALLONNET SOPHAGIENNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **16.09.2021  DE 102021004686**

(43) Veröffentlichungstag der Anmeldung:
**29.03.2023  Patentblatt 2023/13**

(73) Patentinhaber: **Löwenstein Medical Technology S.A.**
**2557 Luxembourg (LU)**

(72) Erfinder: **Kremeier, Peter**
**76149 Karlsruhe (DE)**

(74) Vertreter: **Löwenstein Medical IP**
**Löwenstein Medical Technology**
**GmbH + Co.KG IP Management**
**Kronsaalsweg 40**
**22525 Hamburg (DE)**

(56) Entgegenhaltungen:
EP-A1- 3 520 850        EP-B1- 3 520 850
US-A1- 2009 088 735        US-A1- 2015 038 958

- FRANCESCO MOJOLI ET AL: "In vivo calibration of esophageal pressure in the mechanically ventilated patient makes measurements reliable", CRITICAL CARE, vol. 20, no. 1, 11 April 2016 (2016-04-11), XP055524430, DOI: 10.1186/s13054-016-1278-5
- YAN-LIN YANG ET AL: "Optimal esophageal balloon volume for accurate estimation of pleural pressure at end-expiration and end-inspiration: an in vitro bench experiment", INTENSIVE CARE MEDICINE EXPERIMENTAL, BIOMED CENTRAL LTD, LONDON, UK, vol. 5, no. 1, 2 August 2017 (2017-08-02), pages 1 - 12, XP021247648, DOI: 10.1186/S40635-017-0148-Z
- HOTZ JUSTIN C ET AL: "Measurements Obtained From Esophageal Balloon Catheters Are Affected by the Esophageal Balloon Filling Volume in Children With ARDS", RESPIRATORY CARE, vol. 63, no. 2, 31 October 2017 (2017-10-31), US, pages 177 - 186, XP055921637, ISSN: 0020-1324, DOI: 10.4187/respcare.05685

**Beschreibung**

Wenn kritisch kranke Patienten über einen längeren Zeitraum beatmet werden müssen, wird eine lungenprotektive Beatmungsstrategie benötigt. Das Ziel einer lungenprotektiven Beatmungsstrategie ist es, die Auswirkungen der mechanischen Druck- und Volumenbelastung der Lunge unter Beatmung so gering wie möglich zu halten. Die Adaptation der Beatmung an die individuelle regionale Lungenfunktion und an den Ventilationsbedarf des Patienten muss regelmäßig evaluiert werden, da eine "lungenprotektive" Beatmung die Überlebensrate von Patienten mit akutem Lungenversagen (ARDS) signifikant verbessert.

Es gilt als gesichert, dass das atemsynchrone Kollabieren und Wiedereröffnen von Lungenarealen ("Atelektatrauma") bei ARDS-Patienten einer der Hauptfaktoren für eine beatmungsassoziierte Lungenschädigung ist und einen unabhängigen Risikofaktor für eine höhere Mortalität darstellt. Ein optimal eingestellter PEEP ist eine Grundvoraussetzung für eine lungenprotektive Beatmung, um ein atemzyklisches Öffnen und Kollabieren ("alveolar cycling") zu minimieren. Bei zu niedrigen PEEP-Werten werden Lungenkompartimente durch ein Atelektatrauma, bei zu hohem PEEP und gleichbleibendem "Driving Pressure" durch Überdehnung (Volutrauma) von vorwiegend ventralen Lungenarealen geschädigt.

Für das Ausmaß der mechanischen Stressbelastung auf die Alveolen und damit für die beatmungsassoziierte Lungenschädigung ist nicht der am Respirator eingestellte inspiratorische Plateaudruck (Beatmungsdruck am Ende der Inspiration) entscheidend, sondern der transpulmonale Druck (TPP), definiert als die Differenz von Beatmungsdruck und Pleuraldruck.

Die bettseitige Messung des Ösophagusdrucks (Peso) war in der Vergangenheit wissenschaftlichen Fragestellungen vorbehalten. Zwischenzeitlich erlauben moderne Intensivrespiratoren und neuartige Ballonkatheter dieses Messverfahren minimal invasiv durchzuführen und auf diese Weise wertvolle Informationen über den aktuellen Beatmungsstatus zu liefern. Die Änderungen des Ösophagusdrucks während eines Atemzyklus spiegeln dabei die Änderungen des Pleuraldrucks wieder.

Chiumello et al (Am J Respir Crit Care Med 178;346-355) konnten 2008 zeigen, dass aufgrund der hohen Variabilität des Verhältnisses von Lungenelastance zu Thoraxwandelastance ein am Respirator eingestellter inspiratorischer Plateaudruck in sehr unterschiedlichen Werten für den transpulmonalen Druckgradienten resultierte. Bei Patienten mit erhöhtem Pleuraldruck, z.B. als Folge eines erhöhten intraabdominellen Drucks kann derselbe Inspirationsdruck mit einer geringeren beatmungsassoziierten Lungenschädigung einhergehen als bei Patienten mit niedrigem Pleuraldruck. Wird der transpulmonale Druck negativ, kommt es zur Ausbildung von Atelektasen. Ein entscheidender Aspekt der Wirkung von PEEP ist die Aufrechterhaltung eines positiven end-exspiratorischen transpulmonalen Drucks.

Unter maschineller Beatmung werden als Surrogatparameter für die Berechnung des inspiratorischen bzw. exspiratorischen Alveolardrucks der inspiratorische Plateaudruck (Pplat) bzw. der endexspiratorische Druck (PEEP), für den Pleuraldruck der Ösophagusdruck (Peso) herangezogen.

Der Ösophagusdruck wird über einen kommerziell erhältlichen nasal eingeführten Ballonkatheter, der auch als Ernährungssonde fungiert, gemessen. Die Drucksonde wird an den Drucktransducer des Respirators angeschlossen. Zur Sicherstellung eines validen Messaufbaus und damit eine belastbare ösophagale Druckmessung kommt der Ballonfüllung einen besonderen Stellenwert zu. Wird der Ballon über- oder unterfüllt werden falsch ösophageale Messwerte und damit auch nicht valide transpulmonale Drücke angezeigt.

Das richtige Füllvolumen des Ösophagusballons ist ebenso entscheidend wie die korrekte Lage des Ballons. Da die gemessenen Ösophagusdruckwerte direkt davon abhängen, muss sie patientenindividuell erfolgen. Die von manchen Autoren angegebene Standardfüllung ist wahrscheinlich nicht korrekt und führt zu falschen Messwerten.

Es hat sich herausgestellt, dass das Standardvolumen nicht für jeden Patienten ideal ist und sich das optimale Volumen je nach individuellen Patientenparametern, Lage des Patienten und ähnliches ändern kann. Das Füllvolumen sollte idealerweise so gewählt sein, dass die Durchblutung der Innenwand des Ösophagus nicht beeinträchtigt wird und dennoch zuverlässige Messergebnisse erhalten werden.

Die bettseitige Füllung ist aufwändig und zeitintensiv und bedarf einer herausragenden Praxiserfahrung des medizinischen Personals. Es besteht somit Bedarf an einem Verfahren zur Ermittlung und Aufrechterhaltung eines optimalen Füllvolumens von Ösophagusballons.

Gelöst wird die Aufgabe durch ein System gemäß Anspruch 1.

In den Figuren sind Ausführungsbeispiele des erfindungsgemäßen Systems 100 und des beispielhaften Verfahrens dargestellt. Es zeigen:

Fig. 1 den grundsätzlichen Aufbau eines erfindungsgemäßen Systems.

Fig. 2 einen Ösophagus-Katheter, wobei Fig. 2A eine schematische Seitenansicht des Katheters zeigt und Fig. 2B einen Querschnitt nach Linie S-S durch einen Abschnitt eines Schlauches des Katheters.

Fig. 3 eine schematische Abbildung eines im Ösophagus platzierten Ösophagus-Katheters.

Fig. 4 eine graphische Darstellung eines beispielhaften Druck-Volumen-Verhaltens eines ÖsophagusKatheters *in*

*vitro.*

**Ausführungsbeispiele**

Anhand der nachfolgenden Ausführungsbeispiele ist das beispielhafte Verfahren und das erfindungsgemäße System 100 beschrieben. Weitere Merkmale und Vorteile der vorliegenden Erfindung werden in den nachfolgenden Beschreibungen von Ausführungsbeispielen anhand der Figuren deutlich. Die Erfindung ist nicht auf die dargestellten Ausführungsbeispiele beschränkt. Ein Verfahren als solches fällt nicht unter die Ansprüche.

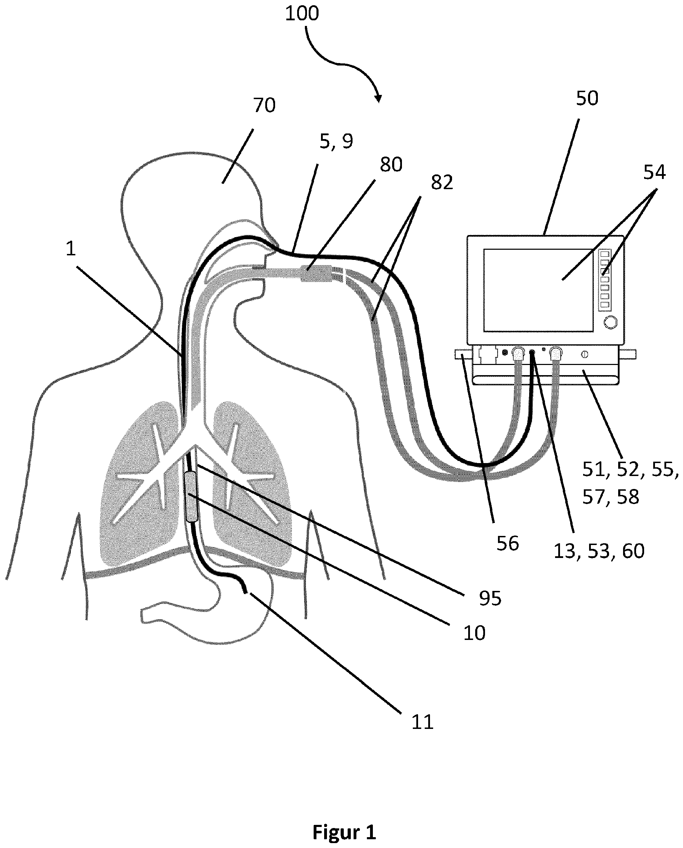

**Figur 1** zeigt den grundsätzlichen Aufbau eines erfindungsgemäßen Systems 100.

Das System 100 umfasst eine Druckermittlungseinrichtung 1, mindestens eine Druckquelle 57 und mindestens eine Leitung 5. Die Druckermittlungseinrichtung ist als medizinischer Katheter 1 ausgebildet. Der Katheter 1 ist bevorzugt ein Ösophaguskatheter. Die mindestens eine Leitung ist als Druckleitung 5 ausgebildet.

Die Druckquelle 57 kann eingerichtet und ausgebildet sein, als Druckquelle und/oder als Saugquelle zu dienen. Die Druckquelle 57 kann Druck applizieren und/oder Druck abbauen. Druck- und Saugquelle können in manchen Ausführungsformen auch als getrennte Einheiten ausgebildet sein. In manchen Ausführungsformen kann die Druckquelle 57 alternativ oder zusätzlich auch als Volumenquelle ausgebildet sein. Die Druckquelle 57 kann somit auch Volumen applizieren und/oder Volumen entnehmen bzw abbauen.

Die Druckquelle 57 ist in ein Beatmungsgerät 50 integriert. Die Druckquelle 57 kann (in nicht vom Schutzumfang umfassten Beispielen) auch ein Anästhesiegerät sein bzw. in ein Anästhesiegerät integriert sein.

Der Katheter 1 umfasst mindestens einen Ösophagusballon 10, der im Folgenden als Ballon 10 bezeichnet wird. Zudem umfasst der Katheter 1 einen Schlauch 9 mit einer Wandung 8 und einem Lumen. Die mindestens eine Druckleitung 5 verläuft mindestens abschnittweise im Lumen und/oder in der Wandung 8 des Schlauches 9. Die Druckleitung 5 ist mit dem Ballon 10 verbunden. Die Druckleitung 5 endet im Ballon 10 des Katheters 1.

Der Katheter 1 umfasst zudem eine pneumatische Schnittstelle 13. Die pneumatische Schnittstelle 13 befindet sich an einem Ende des Katheters 1. Die pneumatische Schnittstelle 13 befindet sich in einem Anwendungszustand außerhalb des Patienten am distalen Ende des Katheters 1. Über die pneumatische Schnittstelle 13 kann der Katheter 1 mit dem Beatmungsgerät 50 verbunden werden. Die pneumatische Schnittstelle 13 kann ein Schaltventil umfassen, das beispielsweise als Dreiwegehahn ausgebildet sein kann (nicht gezeigt).

Um das Beatmungsgerät 50 mit dem Katheter 1 pneumatisch verbinden zu können, kann das Beatmungsgerät 50 einen Katheter-Anschluss 53 aufweisen. Der Katheter 1 kann über die pneumatischen Schnittstelle 13 und den Katheter-Anschluss 53 pneumatisch mit dem Beatmungsgerät 50 verbunden werden.

Sodann verläuft die Druckleitung 5 vom Beatmungsgerät 50 zum Katheter 1. Die Druckleitung 5 beginnt an der Druckquelle 57 des Beatmungsgerätes 50 und endet am Ballon 10 des Katheters 1. Somit kann der Katheter 1 pneumatisch an das Beatmungsgerät 50 angeschlossen werden. Der Ballon 10 des Katheters 1 steht über die Druckleitung 5 und die pneumatische Schnittstelle 13 pneumatisch mit dem Beatmungsgerät 50 in Verbindung. Über die Druckleitung 5 kann der Ballon 10 befüllt werden. Über die Druckleitung 5 kann der Ballon 10 zudem entleert werden. Der Ballon 10 kann mit einem vorbestimmten Druck und/oder Volumen befüllt werden. Der Ballon 10 kann mit einem vorbestimmten Druck und/oder Volumen entleert werden. Der Druck und/oder das Volumen kann vollständig appliziert werden oder stufenweise. Die Entleerung kann aktiv erfolgen, wobei Druck und/oder Volumen aus dem Ballon 10 aktiv abgelassen werden kann. Der Druck und/oder das Volumen kann auch passiv abgelassen werden.

Unter einem Beatmungsgerät 50 sind sämtliche Geräte zu verstehen, die einen Anwender oder Patienten 70 bei der natürlichen Atmung unterstützen und/oder die Beatmung eines Anwenders oder Patienten 70 übernehmen und/oder einer Atemtherapie dienen und/oder anderweitig auf die Atmung eines Anwenders oder Patienten 70 einwirken. Darunter fallen zum Beispiel, aber nicht ausschließlich, CPAP- sowie BiLevel-Geräte, Narkose- bzw. Anästhesiegeräte, Atemtherapiegeräte, klinische, außerklinische oder Notfall-Beatmungsgeräte, Highflow-Therapiegeräte und Hustenmaschinen.

Das Beatmungsgerät 50 kann ein Bedien- und Informationssystem 54 aufweisen. Über das Bedien- und Informationssystem 54 kann der benötigte Druck und/oder Flow und/oder das Volumen eingestellt werden bzw. der aktuelle Druck und/oder Flow und/oder das aktuelle Volumen dargestellt werden. Diese Benutzerinformation kann beispielsweise graphisch visualisiert oder numerisch dargestellt werden.

Das Beatmungsgerät 50 weist die mindestens eine nicht dargestellte Druckquelle 57 sowie eine Steuereinheit 58 auf. Die Druckquelle 57 ist als Druck- und/oder Saugquelle ausgebildet. Die Druckquelle 57 kann einen Druck aufbauen und/oder abbauen bzw. absenken. Die Druckquelle 57 kann auch als Volumenquelle ausgebildet sein. Die Druckquelle 57 kann ein Volumen applizieren und/oder ein Volumen entnehmen bzw abbauen.

Mit der Druckquelle 57 kann der Ballon 10 befüllt und entleert werden. Der Ballon 10 kann über die Druckleitung 5 mit einem Fluid befüllt werden. Der Ballon 10 kann mit einem vorbestimmten Druck und/oder Volumen befüllt werden. Das Fluid ist in der Regel ein Gas, beispielsweise Luft und/oder Sauerstoff und/oder ein Gasgemisch und/oder ein Sauerstoffhaltiges Gasgemisch. Der Ballon 10 ist bevorzugt aus einem flexiblen, Gas- und Wasserundurchlässigem Material gefertigt. Beispielsweise ist der Ballon 10 aus Latex gefertigt.

Zusätzlich zur Druckquelle 57 weist das Beatmungsgerät 50 eine weitere Druckquelle auf, die als Atemgasquelle 55 ausgebildet ist. Die Atemgasquelle 55 kann die Beatmung des Patienten 70 sicherstellen. Die Atemgasquelle 55 kann ein Fluid zur Beatmung bereitstellen. Beispielsweise kann die Atemgasquelle 55 ein Gas und/oder Gasgemisch bereitstellen. Die Atemgasquelle 55 kann Atemgas und/oder Sauerstoff und/oder ein sauerstoffhaltiges Gasgemisch und/oder andere geeignete Gase zur Beatmung bereitstellen. Die Atemgasquelle 55 ist beispielsweise als ein Elektromotor mit Gebläserad oder als Druckgasanschluss ausgebildet. Beispielsweise kann die Atemgasquelle 55 Beatmungsdruck 22 und/oder Flow und/oder Volumen applizieren. Die Steuereinheit 58 kann die Parameter der Beatmung kontrolliert vorgeben und/oder zumindest teilweise assistiert oder adaptiv unter Berücksichtigung von Messsignalen.

Die Druckquelle 57 zur Befüllung und/oder Entleerung des Ballons 10 und die Atemgasquelle 55 zur Bereitstellung des Beatmungsdrucks 22 sind zwei unterschiedliche Druckquellen, die unabhängig voneinander ansteuerbar sind. Die Druckquelle 57 zur Befüllung und/oder Entleerung des Ballons 10 und die Atemgasquelle 55 zur Bereitstellung des Beatmungsdrucks 22 können in manchen Ausführungsformen, die nicht vom Schutzumfang umfasst werden, auch ein und dieselbe Einrichtung sein.

Das Beatmungsgerät 50 kann darüber hinaus zumindest eine Schnittstelle 56 aufweisen, über die eine Datenübertragung möglich ist.

Das System 100 kann neben der Druckermittlungseinrichtung 1, der Druckquelle 50 und der Druckleitung 5 weitere Elemente enthalten.

Insbesondere kann das System 100 über ein Schlauchsystem 82 mit einem Patienteninterface 80 verbunden sein. Über das Patienteninterface 80 kann eine Verbindung vom Beatmungsgerät 50 zu einem Patienten 70 hergestellt werden, um diesen zu beatmen.

Als Patienteninterface 80 ist jegliches Peripheriegerät zu verstehen, welches zur Interaktion mit einem Lebewesen ausgebildet ist. Insbesondere ist das Patienteninterface 80 zu Therapie- und/oder Diagnosezwecken in Verbindung mit dem Beatmungsgerät 50 ausgebildet. Das Patienteninterface 80 kann als Atemmaske ausgebildet sein. Diese Maske kann eine Full-Face-Maske, also Nase und Mund umschließend sein, oder eine Nasenmaske, also eine nur die Nase umschließende Maske. Auch Trachealtuben bzw. -kanülen und sogenannte Nasenbrillen oder Nasenpillow-Masken können als Patienteninterface 80 eingesetzt werden. Das erfindungsgemäße System 100 und Verfahren eignet sich insbesondere auch in Verbindung mit Trachealtuben bzw. -kanülen zur invasiven Beatmung. Das Patienteninterface 80 und das Beatmungsgerät 50 sind bevorzugt über das mindestens eine Schlauchsystem 82 gasleitend miteinander verbunden. Das Schlauchsystem 82 ist bevorzugt flexibel und/oder drehbar ausgebildet. Das Schlauchsystem 82 kann beispielsweise als elastisches Rohr und/oder Schlauch und/oder Schlauchsystem ausgebildet sein.

Das System 100 kann darüber hinaus einen nicht gezeigten Anschluss für eine invasive Blutdruckmessung 86 und/oder zur Temperaturmessung umfassen. Außerdem kann mindestens ein externer Monitor 85 in das System 100 integriert werden. Mehrere externe Monitore 85 sind auch denkbar (nicht gezeigt).

Das System 100 ist eingerichtet und ausgebildet, einen Ösophagus-Druck 20 zumindest zeitweise oder phasenweise zu bestimmen oder zu ermitteln. Der Ösophagus-Druck 20 dient als Surrogatparameter für den Pleuraldruck 21. Die Änderungen des Ösophagus-Drucks 20 während eines Atemzyklus spiegeln die Änderungen des Pleuraldrucks 21 wieder.

Unter maschineller Beatmung werden als Surrogatparameter für die Berechnung des inspiratorischen bzw. exspiratorischen Alveolardrucks der inspiratorische Plateaudruck (Pplat) bzw. der endexspiratorische Druck (PEEP), für den Pleuraldruck der Ösophagus-Druck 20 herangezogen. In manchen Ausführungsformen ist das System zusätzlich ausgebildet und eingerichtet, einen gastralen Druck 24 zu messen, um die gastralen Druckerhöhungen auf die Lunge abschätzen können.

Die Steuereinheit 58 ist beispielsweise zumindest so ausgelegt, dass sie den Ösophagus-Druck 20 bestimmen kann. Zur Bestimmung des Ösophagus-Druckes 20 weist das Beatmungsgerät 50 einen Druckmesseingang 51 und ein Sensormittel 52 auf. Der Druckmesseingang 51 kann beispielsweise pneumatisch, elektronisch und/oder optisch ausgebildet sein. Das Sensormittel kann als Drucksensor 52 ausgebildet sein. Der Drucksensor 52 ist zumindest mittelbar an den Ballon 10 angeschlossen. Mit Hilfe des Drucksensors 52 kann der Ösophagus-Druck 20 bestimmt werden, der über den gasgefüllten Ballon 10 des Katheters 1 erfasst wird. Die Steuereinheit 58 ist beispielsweise eingerichtet und ausgebildet, eine Veränderung des Ösophagus-Druckes 20 zu identifizieren und daraufhin das Beatmungsgerät 50 zur Vorgabe eines Beatmungsparameters anzusteuern.

Bei Überschreiten oder Unterschreiten eines Schwellwertes für den Ösophagus-Druck 20 generiert die Steuereinheit 58 beispielsweise ein Steuersignal für das Beatmungsgerät 50, zur Vorgabe eines inspiratorischen oder exspiratorischen Atemgasdrucks. Bei Überschreiten oder Unterschreiten eines Schwellwertes für den Ösophagus-Druck 20 generiert die Steuereinheit 58 beispielsweise alternativ ein Steuersignal für das Beatmungsgerät 50, zur Beendigung der Vorgabe eines inspiratorischen oder exspiratorischen Atemgasdrucks.

Fig. 1 zeigt schematisch die Anordnung des Beatmungsgerätes 50 in dem System 100. Die Messung des Ösophagus-Druckes 20, hierin auch Peso genannt, basiert auf der Verwendung des Ösophagusballons 10. Der Ösohagus-Druck 20 dient als Surrogatparameter und spiegelt die Änderungen des Pleuraldrucks 21 wider. Der Pleuraldruck 21, auch intrathorakaler Druck genannt, ist der Differenzdruck zwischen dem im Pleuraspalt herrschenden Druck und dem Außendruck.

Aus dem gemessenen Ösophagus-Druck 20 und einem vom Beatmungsgerät 50 vorgegebenen oder gemessenen Beatmungsdruck 22 kann der transpulmonale Druck 23 fortlaufend oder phasenweise rechnerisch bestimmt oder ermittelt werden.

Der transpulmonale Druck 23 ist derjenige Druck, der für die Dehnung der Lunge und der Brustwand erforderlich ist. Der transpulmonale Druck 23 entspricht dem Druckunterschied zwischen den Alveolen und dem Ösophagus. Beispielsweise kann der transpulmonale Druck 23 bei endinspiratorischer oder endexspiratorischer Okklusion bestimmt werden.

Durch die Messung des Ösophagus-Drucks 20 wird somit die Bestimmung des transpulmonalen Drucks 23 ermöglicht. Die fortlaufend oder phasenweise gemessenen oder bestimmten transpulmonalen Drücke 23 erlauben eine Bewertung der mechanischen Druck- und Volumenbelastung unter einer Beatmung, so dass die Beatmung lungenprotektiv adaptiert werden kann. Darüber hinaus hilft die Überwachung des Ösophagus-Drucks 20 beim Erkennen und Behandeln der Ursachen von unwirksamen Patientenanstrengungen.

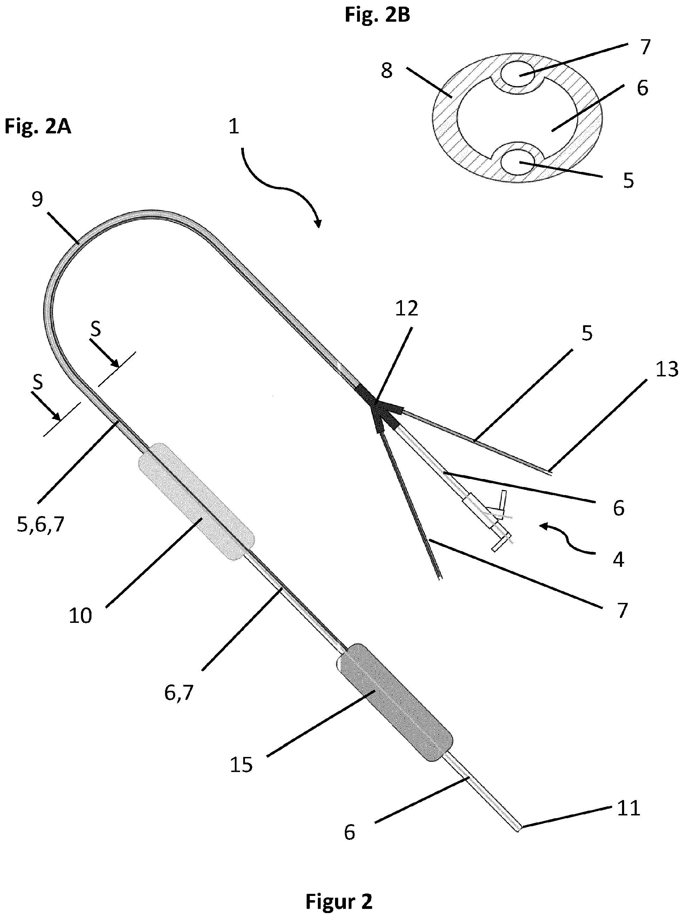

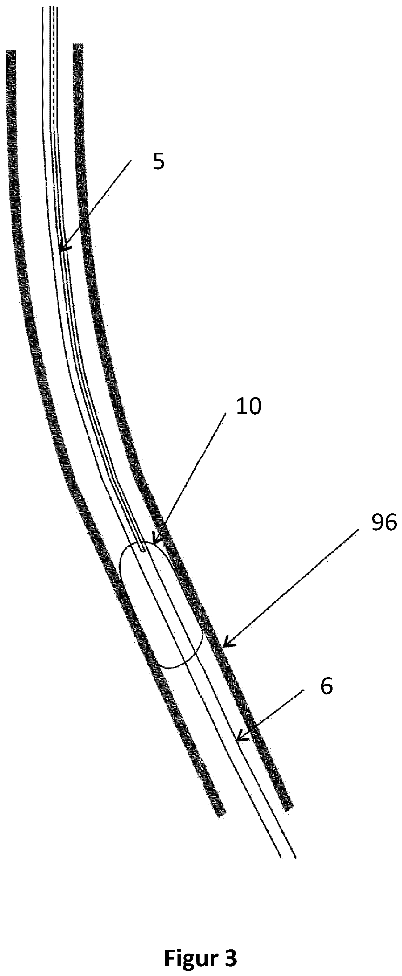

**Figur 2** zeigt einen Ösophagus-Katheter 1, wobei Fig. 2A eine schematische Seitenansicht des Katheters 1 zeigt und Fig. 2B einen Querschnitt nach Linie S-S durch einen Abschnitt des Schlauches 9 des Katheters 1. **Figur 3** zeigt eine schematische Abbildung eines im Ösophagus 95 platzierten ÖsophagusKatheters 1.

Der Katheter 1 ist als Ösophaguskatheter ausgebildet und umfasst den mindestens einen Ballon 10 und den Schlauch 9 sowie mindestens eine Leitung 5.

Die Leitung 5 verläuft zumindest abschnittsweise durch das Lumen des Schlauches 9 und/oder die Schlauchwandung 8. Die Leitung 5 ist als Druckleitung 5 ausgebildet und ist mit dem Ballon 10 verbunden. Die Druckleitung 5 endet im Ballon 10 des Katheters 1. Zusätzlich zur Druckleitung 5 kann der Katheter 1 weitere Leitungen aufweisen. Die ein oder mehreren Leitungen stehen untereinander in der Regel nicht in Kommunikation und sind als gesonderte Leitungen im Lumen des Schlauches 9 und/oder in dessen Wandung 8 angeordnet (siehe Fig. 2B).

In manchen Ausführungsformen kann der Katheter 1 eine Nahrungsleitung 6 und einen Anschluss 4 umfassen. Die Nahrungsleitung 6 erstreckt sich beispielsweise vom Anschluss 4 über die gesamte Länge des Katheters 1 und endet im Ösophagus 95 und/oder im Magen. Bevorzugt endet die Nahrungsleitung 6 im Fundus des Magens. Die Nahrungsleitung 6 und der Anschluss 4 sind eingerichtet und ausgebildet, eine Sondenkosternährung zu ermöglichen. Die Sondenkost kann über den Anschluss 4 in die Nahrungsleitung 6 eingeleitet werden. Die Sondenkost gelangt über die Nahrungsleitung 6 in den Körper des Patienten 70. Die Nahrungsleitung 6 ermöglicht so die direkte Zufuhr von Nahrung in den Magen.

In manchen Ausführungsformen kann der Katheter 1 eine gastrale Druckleitung 7 und einen gastralen Ballon 15 umfassen. Die gastrale Druckleitung 7 kann mit dem gastralen Ballon 15 verbunden sein. Die gastrale Druckleitung 7 endet im gastralen Ballon 15 des Katheters 1. Die gastrale Druckleitung 7 und der gastrale Ballon 15 sind beispielsweise eingerichtet und ausgebildet, den gastralen Druck 24 zu messen. Zu diesem Zweck kann die gastrale Druckleitung 7 mit dem Beatmungsgerät 50 verbunden werden (nicht gezeigt). Der gastrale Druck 24 kann im Zuge der Platzierung des Katheters 1 gemessen werden, um die gastralen Druckerhöhungen auf die Lunge abschätzen können. Optional und/oder zusätzlich kann der Katheter 1 weitere Leitungen aufweisen. Zum Beispiel ist eine Leitung zum Absaugen von Mageninhalt denkbar und/oder eine Leitung zur Zuführung von Medikamenten (nicht gezeigt).

In manchen vorteilhaften Ausgestaltungen des Katheters 1 kann eine Leitung für einen nicht gezeigten Führungsdraht 14 vorgesehen sein. Der Führungsdraht 14 kann den Katheter 1 mechanisch von innen versteifen. Über den Führungsdraht 14 kann das Einführen des Katheters 1 in den Körper des Patienten 70 erleichtert werden.

Der in Fig. 2 beispielhaft dargestellte Katheter 1 weist eine Druckleitung 5, eine Nahrungsleitung 6 und eine gastrale Druckleitung 7 auf (siehe Fig. 2B).

Die Druckleitung 5 endet im Ballon 10. Die gastrale Druckleitung 7 endet im gastralen Ballon 15. Der Katheter 1 umfasst ein Katheterende 11. Die Nahrungsleitung 6 endet am Katheterende 11. Das Katheterende 11 ist in einem Anwendungszustand im oder am Magen oder im Ösophagus 95 angeordnet. Bevorzugt ist das Katheterende 11 im Fundus des Magens angeordnet.

Das Katheterende 11 kann offen ausgebildet sein. Über das offene Katheterende 11 kann beispielsweise Sondenkost aus der Nahrungsleitung 6 in den Ösophagus 95 und/oder Magen entlassen werden. Bevorzugt kann die Sondenkost über das offene Katheterende 11 aus der Nahrungsleitung 6 in den Fundus des Magens entlassen werden.

Es ist auch denkbar, dass Mageninhalt aus dem Magen aufgenommen werden kann und über den Schlauch 9 und/oder über eine gesonderte Leitung im Schlauch 9 oder in der Schlauchwandung 8 aus dem Magen abgeleitet werden kann.

Bei Benutzung des Katheters 1 am Patienten befindet sich ein Teil des Katheters 1 innerhalb des Körpers eines Patienten 70. Der Katheter 1 wird zumindest teilweise in den Ösophagus 95 des Patienten 70 eingeführt. Der Katheter 1 befindet sich in einem Anwendungszustand, wenn sich das Katheterende 11 und zumindest ein Teil des Schlauches 9 sowie der mindestens eine Ballon 10 im Ösophagus 95 und/oder im Magen des Patienten befindet. In einem Anwendungszustand befindet sich das Katheterende 11 bevorzugt im Fundus des Magens und zumindest ein Teil des Schlauches 9 sowie der Ballon 10 im Ösophagus 95 des Patienten.

Der Katheter 1 kann mindestens einen Verteiler 12 umfassen. Der Verteiler 12 befindet sich in einem Anwendungszustand außerhalb des Körpers des Patienten 70. Der Verteiler 12 kann eine Schnittstelle darstellen, an der unterschiedliche Leitungen 5,6,7 zusammen- bzw. auseinandergeführt werden.

Das System 100 kann ein Ventil 60 aufweisen (Fig. 1). Das Ventil 60 ist in einem Anwendungszustand des Katheters 1 bevorzugt außerhalb des Körpers eines Patienten 70 angeordnet. Das Ventil 60 kann bevorzugt im oder am Beatmungsgerät 50 angeordnet sein. Das Ventil 60 kann in manchen Ausführungsformen auch in oder an der Druckleitung 5 angeordnet sein. Das Ventil 60 ist eingerichtet und ausgebildet, ein definiertes Volumen von der Druckquelle 57 des Beatmungsgerätes 50 in den Ballon 10 zu leiten. Über das Ventil 60 kann der Ballon 10 befüllt werden. Das Ventil 60 ist darüber hinaus eingerichtet und ausgebildet, ein definiertes Volumen aus dem Ballon 10 in das Beatmungsgerät 50 zu leiten. Über das Ventil 60 kann der Ballon 10 entleert werden.

Die Befüllung und Entleerung kann bevorzugt über ein Ventil 60 erfolgen. In manchen Ausführungsformen ist es auch denkbar, dass die Befüllung und Entleerung über zwei oder mehr Ventile 60 erfolgt. Die Befüllung und/oder Entleerung erfolgt über die Druckleitung 5. Die Belüftung erfolgt über die Druckleitung 5 vom Beatmungsgerät 50 zum Ballon 10. Die Entleerung erfolgt über die Druckleitung 5 vom Ballon 10 zum Beatmungsgerät 50.

Der Katheter 1 kann zumindest eine der folgenden Funktionen umfassen:

- Messung des Ösophagus-Druckes 20, der den Pleuraldruck 21 widerspiegelt
- Ernährung des Patienten
- Absaugung von Mageninhalt
- Erfassen der Pulsation des Herzens
- Messung des gastralen Druckes 24

Das System 100 ist ausgebildet und eingerichtet, ein Füllvolumen VB von Ösophagusballons 10 zu ermitteln, einzustellen und aufrechtzuerhalten. Das System 100 ist insbesondere ausgebildet und eingerichtet, ein optimales Füllvolumen VBopt von Ösophagusballons 10 zu ermitteln, einzustellen und aufrechtzuerhalten. Das System 100 ist darüber hinaus ausgebildet und eingerichtet, ein minimales Füllvolumen VBa und/oder ein maximales Füllvolumen VBe von Ösophagusballons 10 zu ermitteln. Erfindungsgemäß wird ein Verfahren bereitgestellt zum Ermitteln und Einstellen eines Füllvolumens VB eines Ballons 10 eines Katheters 1, der im Ösophagus 95 eines Lebewesens platziert ist, wobei der Ballon 10 mit einem Fluid befüllt und/oder entleert wird. Dafür können unterschiedliche Ballonvolumina VB appliziert werden und Ballondrücke PB ermittelt werden.

Zur Ermittlung des Ösophagus-Druckes 20 wird das Füllvolumen VB des Ballons 10 bevorzugt derart gewählt, dass der Ballondruck PB dem Ösophagus-Druck 20 entspricht. Der Ösophagus-Druck 20 liefert Informationen über Druckveränderungen im Brustraum zwischen der Lunge und der Brustwand und kann als Surrogatparameter für den Pleuraldruck 21 dienen.

Der Ballondruck PB entspricht dem Ösophagus-Druck 20, wenn das Füllvolumen VB derart gering eingestellt ist, dass die Durchblutung der Innenwand des Ösophagus 95 nicht beeinträchtigt wird und derart hoch eingestellt ist, dass die Ballonwand derart an der Ösophaguswand 96 anliegt, dass zuverlässige Messergebnisse erhalten werden können.

Dementsprechend gibt es für den Ballon 10 eines Ösophaguskatheters 1 ein minimales Füllvolumen VBa und ein maximales Füllvolumen VBe. Der Ballon 10 sollte mindestens mit dem minimalen Füllvolumen VBa und höchstens mit dem maximalen Füllvolumen VBe befüllt sein, damit der Ballondruck PB dem Ösophagus-Druck 20 entspricht und zuverlässige Messergebnisse erhalten werden können. Über das Verfahren kann das minimale Füllvolumen VBa ermittelt werden. Darüber hinaus kann über das Verfahren das maximale Füllvolumen VBe des Ballons 10 ermittelt werden.

Das beispielhafte Verfahren dient insbesondere dazu, ein optimales Füllvolumen VBopt des Ballons 10 zu ermitteln. Die Messung des Ösophagus-Druckes 20 ist besonders valide, wenn der Ballon 10 mit dem optimalen Füllvolumen VBopt befüllt ist. Das optimale Füllvolumen VBopt liegt in einem Bereich zwischen dem minimalen Füllvolumen VBa und dem maximalen Füllvolumen VBe.

Im Folgenden wird das beispielhafte Verfahren beschrieben. Das Verfahren erfolgt bevorzugt mit einem Katheter 1, der in einem Menschen platziert ist. Das Verfahren erfolgt beispielsweise unter kontrollierter Bilevel Beatmung. Alternativ oder ergänzend kann das Verfahren auch unter Spontanatmung erfolgen.

Der Katheter 1 wird im Patienten platziert. Der Ballon 10 des Katheters 1 wird im Ösophagus 95 platziert. Der Ballon 10 des Katheters 1 kann überall im Ösophagus 95 platziert werden. Bevorzugt wird der Ballon 10 im unteren bis mittleren Ösophagus-Drittel platziert (siehe Fig. 3). Besonders bevorzugt wird der Ballon 10 im mittleren Ösophagus-Drittel platziert.

Initial wird der Ballon 10 mit einem vom Hersteller angegebenen Standardvolumen VB0 aufgefüllt. Das Standardvolumen VB0 handelsüblicher Ösophagusballons 10 liegt in der Regel - je nach Größe und Länge des Ballons 10 - zwischen 0,5 ml und 5 ml. Beispielsweise wird der Ballon 10 anfänglich mit einem Standardvolumen VB0 von 3 ml Fluid befüllt.

Anschließend erfolgt eine initiale Druckanpassung. Dafür werden unterschiedliche Ballonvolumina VB appliziert und die entsprechenden Ballondrücke PB gemessen.

Der Ballon 10 wird zunächst wieder entleert. Die Entleerung erfolgt bevorzugt vollständig. Eine vollständige Entleerung liegt vor, wenn sich kein Fluid mehr im Ballon 10 befindet. Bei einer vollständigen Entleerung beträgt das Volumen idealerweise 0 ml.

Es wird mindestens ein Atemhub abgewartet, bevor der Ballon 10 erneut befüllt wird. Bevorzugt wird mehr als ein Atemhub abgewartet. Beispielsweise werden 3 oder 4 Atemhübe abgewartet. Es können auch mehr als 4 Atemhübe abgewartet werden.

Nachdem mindestens ein Atemhub abgewartet wurde, wird der Ballon 10 wieder aufgefüllt. Die Auffüllung erfolgt bevorzugt stufenweise. Eine stufenweise Befüllung bedeutet, dass der Ballon 10 wiederholt mit einem definierten Volumen aufgefüllt wird. Die stufenweise Befüllung erfolgt additiv. Es findet keine Entleerung zwischen den Stufen statt.

Die stufenweise Befüllung und/oder Entleerung erfolgt mit vorbestimmten Volumenstufen Vi. Die Volumenstufen Vi können beispielsweise immer den gleichen Wert aufweisen. In manchen Ausführungsformen können die Volumenstufen Vi auch unterschiedliche Werte aufweisen.

Die Volumenstufen Vi für die einzelnen Befüllungsstufen liegt in einem Bereich von 0,1 ml bis 2 ml. Bevorzugt wird der Ballon 10 in Volumenstufen Vi von 0,2 ml bis 0,8 ml aufgefüllt. Beispielsweise wird der Ballon 10 in Volumenstufen Vi von 0,5 ml aufgefüllt.

Nach jeder Volumenstufe Vi wird mindestens ein Atemhub abgewartet, bevor der Ballon 10 mit einer weiteren Volumenstufe Vi befüllt wird. Bevorzugt wird mehr als ein Atemhub abgewartet. Beispielsweise werden 3 oder 4 Atemhübe abgewartet. Es können auch mehr als 4 Atemhübe abgewartet werden, beispielsweise bis zu 10 Atemhübe oder mehr.

Die Auffüllung erfolgt bis zu einem Endvolumen VB1. Bei dem Endvolumen VB1 ist der Ballon 10 leicht überfüllt bzw. überbläht. Beispielsweise ist der Ballon 10 bei einem Ösophagus-Druck 20 von 30 mbar leicht überfüllt. Beispielsweise wird der Ballon 10 stufenweise bis zu einem Endvolumen VB1 von 10 ml aufgefüllt.

Nach Erreichen eines Ballondruckes PB von beispielsweise 30 mbar und/oder nach Erreichen eines Endvolumens VB1 von beispielsweise 10 ml wird der Ballon wieder entleert.

Die Entleerung erfolgt stufenweise. Eine stufenweise Entleerung bedeutet, dass dem Ballon 10 wiederholt ein definiertes Volumen entnommen wird. Zwischen den einzelnen Stufen der stufenweisen Entleerung findet keine Befüllung statt. Die Entleerung erfolgt vollständig. Die Entleerung erfolgt solange, bis das Volumen des Ballons 10 idealerweise wieder bei 0 ml liegt.

Die stufenweise Entleerung erfolgt mit vorbestimmten Volumenstufen Vi. Die Volumenstufen Vi für die stufenweise Entleerung liegt in einem Bereich von 0,1 ml bis 2 ml. Bevorzugt wird der Ballon 10 in Volumenstufen Vi von 0,2 ml bis 0,8 ml entleert. Beispielsweise wird der Ballon 10 in Volumenstufen Vi von 0,5 ml entleert. Das Volumen Vi der Entleerungsstufen ist bevorzugt gleich dem Volumen Vi der Befüllungsstufen. Das Volumen Vi der Entleerungsstufen kann in manchen Ausführungsformen des Verfahrens größer oder kleiner sein als das Volumen Vi der Befüllungsstufen.

Nach jeder Entleerungsstufe wird mindestens ein Atemhub abgewartet, bevor der Ballon 10 mit einer weiteren Volumenstufe Vi entleert wird. Bevorzugt wird mehr als ein Atemhub abgewartet. Beispielsweise werden 3 oder 4 Atemhübe abgewartet. Es können auch mehr als 4 Atemhübe abgewartet werden.

Die Anzahl der abzuwartenden Atemhübe nach jeder Entleerungsstufe ist bevorzugt gleich der Anzahl der abzuwartenden Atemhübe nach jeder Befüllungsstufe. Es können jedoch auch mehr oder weniger Atemhübe nach jeder Entleerungsstufe abgewartet werden, als nach jeder Befüllungsstufe.

Nach den einzelnen Befüllungsstufen und/oder Entleerungsstufen wird jeweils mindestens ein Atemhub abgewartet, um Schwingungsartefakte des Ballons 10 zu reduzieren.

Der Ballondruck PB kann während der Befüllung und/oder Entleerung des Ballons 10 kontinuierlich ermittelt werden. Der Ballondruck PB kann für jede einzelne Volumenstufe Vi der Befüllung und/oder Entleerung ermittelt werden. Beispielsweise wird der Ballondruck PB mindestens bei jeweils einem Atemhub je Volumenstufe Vi der Befüllung und/oder Entleerung ermittelt. Bevorzugt wird der Ballondruck PB für alle Atemhübe ermittelt.

Der Ballondruck PB wird bevorzugt am Ende der Inspiration (Pmax) und am Ende der Exspiration (Pmin) ermittelt.

Die Druckwerte Pmin und Pmax können analysiert werden, um das minimale Füllvolumen VBa und das maximalen Füllvolumen VBe zu ermitteln. Die Werte Pmin und Pmax können darüber hinaus analysiert werden, um das optimale Füllvolumen VBopt zu ermitteln. Der Ballondruck PB kann in Abhängigkeit vom Füllvolumen VB analysiert werden.

Die ermittelten Ballondrücke PB je Volumenstufe Vi der Befüllung und/oder Entleerung können einzeln ausgewertet werden oder gemittelt werden. Beispielsweise wird zum einen der Mittelwert von Pmax je Volumenstufe Vi ermittelt und zum anderen der Mittelwert von Pmin je Volumenstufe Vi. Für die Mittelung können alle oder nur einige ausgewählte Ballondrücke PB verwendet werden. Alternativ oder ergänzend zum Mittelwert können aus den Messwerten der Ballondrücke PB auch Mediane, Perzentile, Ableitungen, Häufigkeitsverteilungen oder ähnliches ermittelt werden und der weiteren Berechnung zugrunde gelegt werden.

Die Messwerte Pmax und Pmin, die bei oben genannten Vorgehen erhalten werden, können analysiert werden. Vorteilhaft ist die Ermittlung einer Druckdifferenz ΔPB zwischen Pmax und Pmin. Diese Druckdifferenz ΔPB wird auch als Delta-Peso bezeichnet.

Die Druckdifferenz ΔPB wird bevorzugt für jede Volumenstufen Vi der Befüllung und/oder Entleerung ermittelt. Insbesondere können die Druckdifferenzen ΔPB zwischen Pmax und Pmin von jeweils identischen Füllungs- und/oder Entleerungs-Stufen ermittelt werden. Die Veränderung der Druckdifferenzen ΔPB zwischen Pmax und Pmin sind ein Indikator dafür, wann der Ballondruck PB dem Ösophagus-Druck 20 entspricht.

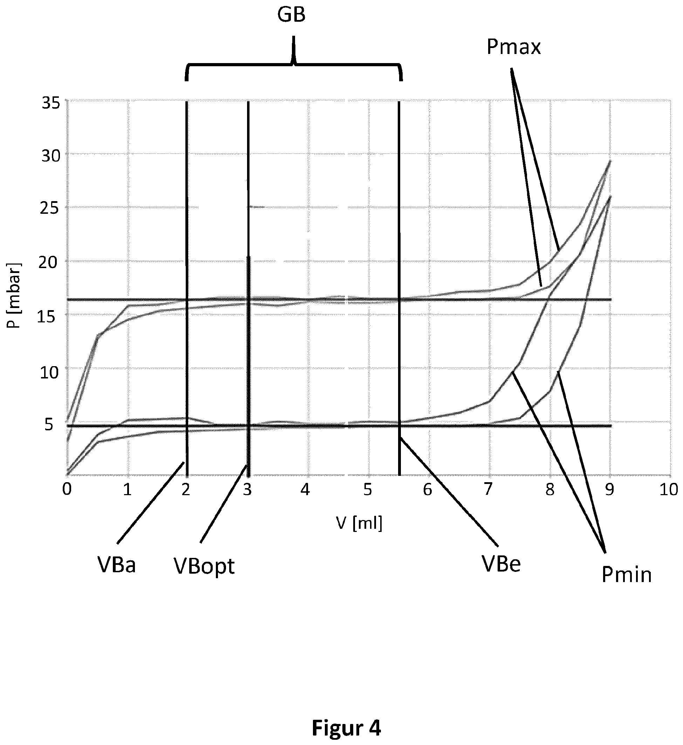

Die ermittelten Messwerte können analysiert und beispielsweise graphisch dargestellt werden. Analysiert werden kann beispielsweise der Ballondruck PB am Ende der Exspiration Pmin und/oder der Ballondruck PB am Ende der Inspiration Pmax in Abhängigkeit vom Füllvolumen VB. Die Messwerte können beispielsweise in einem Achsendiagramm dargestellt werden (siehe Fig. 4).

**Figur 4** zeigt eine graphische Darstellung eines beispielhaften Druck (P) - Volumen (V) - Verhaltens eines Ösophagus-Katheters 1 *in vitro. In* vitro-Untersuchungen von Ösophaguskathetern 1 zeigen eine typische Hysterese bezüglich ihres Druck - Volumen Verhaltens.

Auf der X-Achse ist das Volumen V, nämlich das Füllvolumen VB des Ballon 10 in ml aufgetragen. Auf der Y-Achse ist der Druck P, nämlich der Ballondruck PB in mbar aufgetragen.

Aus Fig. 4 werden Druckdifferenzen ΔPB zwischen Pmax und Pmin ersichtlich. Diese Druckdifferenzen ΔPB wird hierin auch als Delta-Peso bezeichnet. Das Delta-Peso beschreibt den Druckunterschied zwischen Pmax und Pmin in Abhängigkeit vom Füllvolumen VB.

Aus Fig. 4 ist ersichtlich, dass die Kurven von Pmin und Pmax im Bereich von 2 ml bis 5,5 ml Füllvolumen VB nahezu parallel verlaufen. Es bildet sich ein Plateau, also ein Bereich, in dem Pmin und Pmax sich zueinander nicht wesentlich verändern.

Die Veränderung der Druckdifferenzen ΔPB zwischen Pmax und Pmin sind ein Indikator dafür, wann der Ballondruck PB dem Ösophagus-Druck 20 entspricht. Die Druckdifferenz ΔPB ist die Differenz zwischen Pmax und Pmin.

Die Druckdifferenz ΔPB kann beispielsweise wie folgt berechnet werden: ΔPB = Pmax - Pmin

Die Druckdifferenz ΔPB von einer Volumenstufe Vi der stufenweisen Befüllung und/oder Entleerung zur nächsten Volumenstufe Vi+1 kann beispielsweise als prozentualer Wert oder Indexwert ermittelt werden, um eine relative Druckdifferenz rΔPB zu ermitteln.

Die Volumenstufe Vi ist hierbei das Volumen der i-ten Volumenstufe (Befüllungs- oder Entleerungsstufe). Die Volumenstufe Vi+1 ist das Volumen der darauffolgenden Volumenstufe (Befüllungs- oder Entleerungsstufe). Die Druckdifferenzen ΔPB von aufeinander folgenden Volumenstufen (Vi, Vi+1) werden miteinander in Beziehung gesetzt, so dass eine prozentuale Abweichung ermittelt werden kann.

Die relative Druckdifferenz rΔPB kann beispielsweise ermittelt werden unter Hinzuziehung folgender Formel:

$$r\Delta PB = \left| \frac{\Delta PB(Vi+1) - \Delta PB(Vi)}{\Delta PB(Vi+1)} \right| * 100\%$$

Auf Grundlage der relativen Druckdifferenz rΔPB kann ein Grenzbereich GB identifiziert werden. Innerhalb des Grenzbereiches GB verlaufen die Verlaufskurven von Pmin und Pmax nahezu konstant. Innerhalb des Grenzbereiches GB weichen Pmin und Pmax der aufeinanderfolgenden Volumenstufen Vi nicht wesentlich voneinander ab. Zudem verlaufen die Kurven von Pmin und Pmax innerhalb des Grenzbereiches GB annähernd parallel zueinander. Innerhalb des Grenzbereiches GB ist die Druckdifferenz ΔPB somit annähernd konstant (siehe Fig. 4).

Der Grenzbereich GB liegt deutlich unter 100%, bevorzugt unterhalb von 50%, besonders bevorzugt unterhalb von 25%. In einem konkreten Ausführungsbeispiel liegt der Grenzbereich GB in einem Bereich von 0 bis einschließlich 10% (nicht gezeigt).

Ist die relative Druckdifferenz rΔPB größer als der Grenzbereich GB, so liegt der Ballondruck PB außerhalb des Ösophagus-Druckes 20 und ist für eine Messung des Ösophagus-Druckes 20 nicht geeignet. Liegt die relative Druckdifferenz rΔPB innerhalb des Grenzbereiches GB, entspricht der Ballondruck PB dem Ösophagus-Druck 20 und ist für eine Messung des Ösophagus-Druckes 20 geeignet.

Somit kann aus einer stufenweisen Befüllung und Entleerung des Ballons 10 das minimale Füllvolumen VBa und das maximale Füllvolumen VBe abgeleitet werden.

Das minimale Füllvolumen VBa liegt dann vor, wenn nach einer Volumenstufe Vi die relativen Druckdifferenz rΔPB erstmals innerhalb des Grenzbereiches GB liegt. Das minimale Füllvolumen VBa liegt also dann vor, wenn sie den minimalen Wert des Grenzbereichs GB nicht unterschreitet.

Das minimale Füllvolumen VBa liegt somit am Beginn, von dem ab Pmax und Pmin nahezu parallel verlaufen.

Die relative Druckdifferenz r∆PB beim minimalen Füllvolumen VBa ist durch das obere Ende des Grenzbereiches GB definiert. In einer konkreten Ausführungsform kann die relative Druckdifferenz r∆PB beim minimalen Füllvolumen VBa bei 10 % liegen.

Das maximale Füllvolumen VBe liegt bei der Volumenstufe Vi vor, bei der die relativen Druckdifferenz r∆PB letztmalig innerhalb des Grenzbereiches GB liegt. Das maximale Füllvolumen VBe liegt also dann vor, wenn sie den minimalen Wert des Grenzbereichs GB nicht überschreitet.

Das maximale Füllvolumen VBe liegt somit am Ende, von dem ab Pmax und Pmin letztmalig nahezu parallel verlaufen und konstant verlaufen. Die relative Druckdifferenz r∆PB beim maximalen Füllvolumen VBa ist ebenso durch das obere Ende des Grenzbereiches GB definiert. In einer konkreten Ausführungsform kann die relative Druckdifferenz r∆PB beim maximalen Füllvolumen VBe bei 10 % liegen.

Die relative Druckdifferenz r∆PB bei Füllvolumina, die über dem maximalen Füllvolumen VBe liegen, befinden sich außerhalb des Grenzbereiches GB.

Füllvolumina, die kleiner als das minimale Füllvolumen VBa und/oder größer als das maximale Füllvolumen VBe sind, sind für eine Messung des Ösophagus-Druckes 20 nicht geeignet.

Füllvolumina, die größer als das minimale Füllvolumen VBa und/oder kleiner als das maximale Füllvolumen VBe sind, sind für eine Messung des Ösophagus-Druckes 20 geeignet.

Das optimales Füllvolumen VBopt liegt in dem Bereich zwischen dem minimalen Füllvolumen VBa und dem maximalen Füllvolumen VBe. In manchen Ausführungsformen kann das optimales Füllvolumen VBopt auch gleich dem minimalen Füllvolumen VBa sein oder gleich dem maximalen Füllvolumen VBe sein.

In einer bevorzugten Ausführungsform ist das optimale Füllvolumen VBopt größer als das minimale Füllvolumen VBa und kleiner als das maximale Füllvolumen VBe.

Wenn der Ballon 10 mit dem optimalen Füllvolumen VBopt befüllt ist, weist der Ballon 10 eine optimale Anpassung an den Ösophagus 95 auf. Die Ösophaguswand 96 wird durch den mit dem optimalen Füllvolumen VBopt befüllten Ballon 10 nicht negativ beeinflusst. Zudem liefert der Ballon 10 zuverlässige, valide, reproduzierbare Messwerte, wenn er mit dem optimalen Füllvolumen VBopt befüllt ist. Die Druckänderungen im Ballon 10 spiegeln den Ösophagus-Druck 20 und somit den Pleuraldruck 21 optimal wider. Der Pleuraldruck 21 spiegelt wiederrum den Druck in der Lunge wider.

In manchen Ausführungsformen liegt das optimale Füllvolumen VBopt mehr als 10 % über dem minimalen Füllvolumen VBa und weniger als 80 % unter dem maximalen Füllvolumen VBe. Bevorzugt liegt das optimale Füllvolumen VBopt mehr als 20 % über dem minimalen Füllvolumen VBa und weniger als 50 % unter dem maximalen Füllvolumen VBe. In einem konkreten Ausführungsbeispiel liegt das optimale Füllvolumen VBopt 30 % über dem minimalen Füllvolumen VBa und 70 % unter dem maximalen Füllvolumen VBe.

Das optimale Füllvolumen VBopt kann beispielsweise mit folgender Formel berechnet werden:

$$VBopt = VBa + \frac{(VBe - VBa)}{3}$$

Aus Fig. 4 wird ersichtlich, dass das minimale Füllvolumen VBa in diesem konkreten Ausführungsbeispiel beispielsweise ca. 2 ml beträgt und das maximale Füllvolumen VBe beispielsweise ca. 5,5 ml beträgt. Zwischen dem minimalen Füllvolumen VBa und dem maximalen Füllvolumen VBe liegen Pmin und Pmax in einem nahezu konstanten Bereich.

Bei einem minimalen Füllvolumen VBa von beispielsweise 2 ml und einem maximalen Füllvolumen VBe von beispielsweise 5,5 ml beträgt das optimale Füllvolumen VBopt somit beispielsweise ca. 3,17 ml.

Sind optimales Füllvolumen VBopt und das zu Beginn eigestellte Standardvolumen VB0 nicht gleich und wird die initiale Druckanpassung nicht mehr durchgeführt, so kann der Ballondruck PB korrigiert werden. Der Ballon 10 kann mit dem ermittelten optimalem Füllvolumen VBopt eingestellt werden.

Alternativ und ergänzend kann eine Druckkorrektur des optimalen Füllvolumens VBopt vorgenommen werden. Die Druckkorrektur korrigiert das optimale Füllvolumen VBopt unter Einbeziehung der Ösophaguscompliance Ces.

Die Ösophaguscompliance Ces ist die elastische Volumendehnbarkeit des Ösophagus 95. Dadurch, dass die Ösophaguswand 96 eine Dehnbarkeit aufweist, wird der Ballondruck PB bei einer Vergrößerung des Ballons 10 größer und bei einer Verkleinerung des Ballon 10 kleiner. In vivo kann somit anstelle eines Plateaus ein linear ansteigender Druckverlauf entstehen, der durch die Ösophaguscompliance Ces verursacht wird (nicht gezeigt). Die Ösophaguscompliance Ces des Ösophagus 95 ist in vivo nicht einheitlich und kann je nach Abschnitt des Ösophagus 95 variieren. Für das erfindungsgemäße Verfahren kann die Ösophaguscompliance Ces beispielhaft als konstant vorausgesetzt werden.

In manchen Ausführungsformen wird die Ösophaguscompliance Ces in regelmäßigen Abständen neu ermittelt. Es kann vorgesehen sein, dass die Ösophaguscompliance Ces ergänzend oder alternativ zumindest dann neu ermittelt wird, wenn festgestellt wird, dass das Füllvolumen VB des Ballons 10 außerhalb des Grenzbereiches GB liegt bzw. sich der Grenzbereich GB so ändert, dass das Füllvolumen VB zumindest einmalig außerhalb des Grenzbereiches GB liegt.

Es besteht ein Zusammenhang zwischen der Veränderung des Füllvolumens ∆VB und der Veränderung des Ballondruckes ∆PB durch die Ösophaguscompliance Ces. Die Wandverdrängung des Ösophagus 95 durch den Ballon 10 kann mit folgender Formel abgebildet werden: ∆VB = Ces * ∆PB

Für den Volumenbereich, der für eine Messung des Ösophagus-Druckes 20 in Frage kommt, wird die Ösophaguscompliance Ces als konstant vorausgesetzt.

Um die Ösophaguscompliance Ces zu ermitteln, wird das minimale Füllvolumen VBa und das maximale Füllvolumen VBe herangezogen sowie die Drücke am Ende der Exspiration Pmin. PBEa ist der Ballondruck am Ende der Exspiration beim minimalen Füllvolumen VBa. PBEe ist der Ballondruck am Ende der Exspiration beim maximalen Füllvolumen VBe.

Die Ösophaguscompliance Ces kann beispielsweise anhand folgender Formel berechnet werden:

$$Ces = \frac{VBe - VBa}{PBEe - PBEa}$$

Eine genauere Berechnung wird erreicht, wenn eine lineare Regression über den Volumenbereich zwischen dem minimalen Füllvolumen VBa und dem maximalen Füllvolumen VBe durchgeführt wird. Gemäß der Formel ∆VB = Ces * ∆PB kann die Druckkorrektur wie folgt angewendet werden.

Für den Fall, dass das optimales Füllvolumen VBopt größer als das Standardvolumen VB0 ist, kann gelten:

$$\Delta PB = Ces(VBopt - VB0)$$

Für den Fall, dass das optimales Füllvolumen VBopt kleiner als das Standardvolumen VB0 ist, kann gelten:

$$\Delta PB = Ces(VB0 - VBopt)$$

Über das erfindungsgemäße Verfahren kann das optimale Füllvolumen VBopt sowie ein korrigiertes optimales Füllvolumen VBoptkorr ermittelt werden. Somit kann der Ballon 10 mit dem optimalen Füllvolumen VBopt befüllt werden. Bevorzugt kann der Ballon 10 mit dem korrigierten optimalen Füllvolumen VBoptkorr befüllt, der die Ösophaguscompliance Ces berücksichtigt.

Alternativ oder ergänzend kann das Verhältnis zwischen appliziertem Füllvolumen VB und dem Ballondruck PB überwacht werden, um eine mögliche Leckage zu detektieren und zu kompensieren.

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich des beiliegenden Anspruchs nicht verlassen wird. Die vorliegende Offenbarung schließt sämtliche Kombinationen der vorgestellten Einzelmerkmale mit ein.

**Bezugszeichenliste**

| 1 | Katheter |
|---|---|
| 4 | Anschluss |
| 5 | Druckleitung |
| 6 | Nahrungsleitung |
| 7 | Gastrale Druckleitung |
| 8 | Schlauchwandung |
| 9 | Schlauch |
| 10 | Ballon |
| 11 | Katheterende |
| 12 | Verteiler |

(fortgesetzt)

| 13 | Pneumatische Schnittstelle |
|---|---|
| 14 | Führungsdraht |
| 15 | Gastraler Ballon |
| 20 | Ösophagus-Druck (Peso) |
| 21 | Pleuraldruck (intrathorakaler Druck) |
| 22 | Beatmungsdruck (PAW) |
| 23 | Transpulmonaler Druck (TPP) |
| 24 | Gastraler Druck |
| 50 | Beatmungsgerät |
| 51 | Druckmesseingang |
| 52 | Drucksensor |
| 53 | Katheter-Anschluss |
| 54 | Bedien-und Informationssystem |
| 55 | Atemgasquelle |
| 56 | Schnittstelle |
| 57 | Druckquelle |
| 58 | Steuereinheit |
| 60 | Ventil |
| 70 | Patient |
| 80 | Patienteninterface |
| 82 | Schlauchsystem |
| 85 | Externer Monitor |
| 86 | Anschluss invasive Blutdruckmessung |
| 95 | Ösophagus |
| 96 | Ösophaguswand |
| 100 | System |
| Ces | Ösophaguscompliance |
| GB | Grenzbereich |
| P | Druck |
| PB | Ballondruck |
| Pmax | Ballondruck am Ende der Inspiration |
| Pmin | Ballondruck am Ende der Exspiration |
| ΔPB | Druckdifferenz zwischen Pmax und Pmin / Delta-Peso |
| rΔPB | relative Druckdifferenz |
| S | Schnittebene |
| V | Volumen |
| VB | Füllvolumen |
| VB0 | Standardvolumen |
| VB1 | Endvolumen |
| Vi | Volumenstufe (Befüllungsstufe / Entleerungsstufe) |

(fortgesetzt)

| VBa | Minimales Füllvolumen |
|---|---|
| VBe | Maximales Füllvolumen |
| VBopt | Optimales Füllvolumen |
| VBoptkorr | Korrigiertes optimales Füllvolumen |
| ΔVB | Volumendifferenz |

**Patentansprüche**

1. System (100) zum Ermitteln und Einstellen eines Füllvolumens (VB) eines Ballons (10) eines Katheters (1), der im Ösophagus eines Lebewesens platziert ist, umfassend

- den Katheter (1)
- mindestens eine Druckquelle (57) mit einer Steuereinheit (58)
- mindestens eine Leitung (5), über die der Katheter (1) und die Druckquelle (57) pneumatisch miteinander in Verbindung stehen
- mindestens einem Sensormittel, welches als Drucksensor (52) ausgebildet ist

wobei das System (100) eingerichtet und ausgebildet ist, ein
Verfahren zum Ermitteln und Einstellen eines Füllvolumens (VB) eines Ballons (10) eines Katheters (1), der im Ösophagus (95) eines Lebewesens platziert ist, wobei der Ballon (10) mit einem Fluid befüllt und/oder entleert wird, umfassend die folgenden Verfahrensschritte

- Stufenweises Befüllen und/oder Entleeren des Ballons (10) mit mindestens zwei Volumenstufen (Vi) mittels der Steuereinheit (58)
- Bestimmen, mittels der Steuereinheit (58), einer Druckdifferenz (ΔPB) zwischen einem Druck am Ende einer Exspiration (Pmin) und einem Druck am Ende einer Inspiration (Pmax) für mindestens zwei Volumenstufen (Vi)
- Bestimmen, mittels der Steuereinheit (58), einer relativen Druckdifferenz (rΔPB) zwischen (Pmin) und (Pmax)
- Festlegen, mittels der Steuereinheit (58), eines Grenzbereiches (GB) anhand der relativen Druckdifferenz (rΔPB)
- Ermitteln, mittels der Steuereinheit (58), eines optimalen Füllvolumens (VBopt) unter Berücksichtigung des Grenzbereiches (GB) auszuführen,
**dadurch gekennzeichnet, dass** die Druckquelle (57) und die Steuereinheit (58) in ein Beatmungsgerät (50) integriert sind, wobei
das Beatmungsgerät (50) zusätzlich zu der Druckquelle (57) noch eine weitere Druckquelle aufweist, die als Atemgasquelle (55) ausgebildet ist, welche die Beatmung eines Patienten sicherstellt, wobei
die Druckquelle (57) zur Befüllung und/oder Entleerung des Ballons (10) ausgebildet ist und die Atemgasquelle (55) zur Bereitstellung eines Beatmungsdrucks (22) ausgebildet ist und beide Druckquellen (55, 57) unterschiedliche Druckquellen sind, die unabhängig voneinander ansteuerbar sind.

**Claims**

1. A system (100) for ascertaining and adjusting a filling volume (VB) of a balloon (10) of a catheter (1) which is placed in the esophagus of a living being, comprising

- the catheter (1)
- at least one pressure source (57) having a control unit (58)
- at least one line (5) via which the catheter (1) and the pressure source (57) are pneumatically interconnected
- at least one sensor means which is designed as a pressure sensor (52)

wherein the system (100) is configured and designed to carry out a method for ascertaining and adjusting a filling volume (VB) of a balloon (10) of a catheter (1) which is placed in the esophagus (95) of a living being, wherein the balloon (10) is filled with a fluid and/or emptied, comprising the following method steps

- filling and/or emptying the balloon (10) stepwise with at least two volume levels (Vi) by means of the control unit (58)
- determining, by means of the control unit (58), a pressure difference ($\Delta$PB) between a pressure at the end of an expiration (Pmin) and a pressure at the end of an inspiration (Pmax) for at least two volume levels (Vi)
- determining, by means of the control unit (58), a relative pressure difference (r$\Delta$PB) between (Pmin) and (Pmax)
- setting, by means of the control unit (58), a limit range (GB) based on the relative pressure difference (r$\Delta$PB)
- ascertaining, by means of the control unit (58), an optimum filling volume (VBopt) taking into account the limit range (GB),
**characterized in that** the pressure source (57) and the control unit (58) are integrated in a ventilator (50), wherein the ventilator (50) has, in addition to the pressure source (57), a further pressure source which is designed as a breathing gas source (55) that ensures ventilation of a patient, wherein
the pressure source (57) is designed to fill and/or empty the balloon (10) and the breathing gas source (55) is designed to provide a ventilation pressure (22) and both pressure sources (55, 57) are different pressure sources which can be actuated independently of one another.

## Revendications

1. Système (100) destiné à la détermination et au réglage d'un volume de remplissage (VB) d'un ballon (10) d'un cathéter (1), lequel est placé dans l'œsophage d'un être vivant, comportant

   - le cathéter (1)
   - au moins une source de pression (57) avec une unité de commande (58)
   - au moins une ligne (5) par le biais de laquelle le cathéter (1) et la source de pression (57) sont reliés pneumatiquement l'un à l'autre
   - au moins un moyen de capteur, lequel est conçu comme un capteur de pression (52)

   dans lequel le système (100) est configuré et conçu pour mettre en œuvre un procédé de détermination et de réglage d'un volume de remplissage (VB) d'un ballon (10) d'un cathéter (1), lequel est placé dans l'œsophage (95) d'un être vivant, dans lequel le ballon (10) est rempli avec un fluide et/ou vidé, comportant les étapes de procédé suivantes :

   - remplissage et/ou vidage progressifs du ballon (10) avec au moins deux niveaux de volume (Vi) au moyen de l'unité de commande (58)
   - détermination, au moyen de l'unité de commande (58), d'une différence de pression ($\Delta$PB) entre une pression à la fin d'une expiration (Pmin) et une pression à la fin d'une inspiration (Pmax) pour au moins deux niveaux de volume (Vi)
   - détermination, au moyen de l'unité de commande (58), d'une différence de pression relative (r$\Delta$PB) entre (Pmin) et (Pmax)
   - définition, au moyen de l'unité de commande (58), d'une zone limite (GB) à l'aide de la différence de pression relative (r$\Delta$PB)
   - détection, au moyen de l'unité de commande (58), d'un volume de remplissage optimal (VBopt) en tenant compte de la zone limite (GB),
   **caractérisé en ce que** la source de pression (57) et l'unité de commande (58) sont intégrées dans un appareil respiratoire (50), dans lequel
   en plus de la source de pression (57), l'appareil respiratoire (50) présente également une autre source de pression conçue en tant que source de gaz respiratoire (55), laquelle assure la ventilation d'un patient, dans lequel la source de pression (57) est conçue pour remplir et/ou vider le ballon (10) et la source de gaz respiratoire (55) est conçue pour fournir une pression de ventilation (22), et les deux sources de pression (55, 57) sont des sources de pression distinctes, lesquelles peuvent être actionnées indépendamment l'une de l'autre.

**Figur 1**

**Fig. 2B**

**Fig. 2A**

**Figur 2**

Figur 3

**Figur 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **CHIUMELLO et al.** *Am J Respir Crit Care Med*, vol. 178, 346-355